(19) Europäisches Patentamt
European Patent Office
Office européen des brevets

(11) **EP 4 082 582 B1**

(12) **EUROPEAN PATENT SPECIFICATION**

(45) Date of publication and mention
of the grant of the patent:
**15.10.2025 Bulletin 2025/42**

(51) International Patent Classification (IPC):
***A61L 2/10*** *(2006.01)*     ***A61L 9/20*** *(2006.01)*
***A61L 2/24*** *(2006.01)*

(21) Application number: **22168706.4**

(22) Date of filing: **19.04.2022**

(52) Cooperative Patent Classification (CPC):
**A61L 2/10; A61L 2/24; A61L 9/20;** A61L 2202/11;
A61L 2202/16; A61L 2202/25; A61L 2209/111

(54) **SYSTEMS AND METHODS FOR REDUCING ARCING OF ULTRAVIOLET LAMPS**

SYSTEME UND VERFAHREN ZUR VERMINDERUNG DER LICHTBOGENBILDUNG VON
ULTRAVIOLETTLAMPEN

SYSTÈMES ET PROCÉDÉS DE RÉDUCTION DE LA FORMATION D'ARCS DE LAMPES À
ULTRAVIOLETS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.04.2021 US 202163177991 P**

(43) Date of publication of application:
**02.11.2022 Bulletin 2022/44**

(73) Proprietor: **The Boeing Company
Arlington, VA 22202 (US)**

(72) Inventors:
• **Brockschmidt JR, Arthur E.
CHICAGO, 60606-1596 (US)**
• **Childress, Jamie J.
CHICAGO, 60606-1596 (US)**

(74) Representative: **Arnold & Siedsma
Bezuidenhoutseweg 57
2594 AC The Hague (NL)**

(56) References cited:
WO-A1-2012/129243     WO-A1-2014/204731
US-A1- 2015 262 780     US-B1- 6 787 782

## Description

FIELD OF THE DISCLOSURE

Examples of the present disclosure generally relate to systems and methods for reducing arcing of ultraviolet (UV) lamps, such as may be used to sanitize structures and areas within vehicles, such as commercial aircraft.

BACKGROUND OF THE DISCLOSURE

Vehicles such as commercial aircraft are used to transport passengers between various locations. Systems are currently being developed to disinfect or otherwise sanitize surfaces within aircraft, for example, that use ultraviolet (UV) light.

A known UV lamp includes an enclosed tube including UV light emitters. The tube is connected to electrodes, which are exposed to an ambient environment. Arcing between electrodes occurs at a known breakdown voltage. Further, the breakdown voltage within air decreases if the electrodes are exposed to ionizing radiation. Moreover, when ultraviolet light, such as emitted by the UV light emitters, is present, air within the environment is already ionized. As such, an electric field need not be as high in order for arcing to occur. In short, UV light decreases the breakdown voltage, thereby increasing a potential for arcing.

As an aircraft ascends to higher altitudes, the ambient pressure decreases. When UV lamps are operated at such altitudes, the breakdown voltage consequently decreases. Certain UV lamps may be susceptible to arcing at pressures within an internal cabin of an aircraft. In order to mitigate potential arcing, the UV lamp may therefore not be operated at certain pressures within the internal cabin. As such, the UV lamp may not be able to sanitize various components as desired. US6787782 B1 mentions a circuitry with temperature compensation and current control.

SUMMARY OF THE DISCLOSURE

A need exists for a system and a method for reducing potential arcing of a UV lamp. Further, a need exists for system and method for adapting operation of a UV lamp at various altitudes and air pressures.

With those needs in mind, certain examples provide a system including an ultraviolet (UV) lamp including one or more UV light emitters coupled to electrodes. The UV light emitters are configured to emit UV light within an environment. A power source is coupled to the UV lamp. The power source is configured to supply power to the UV lamp. A pressure sensor is configured to detect an ambient air pressure within the environment. A temperature sensor is configured to detect an ambient air temperature within the environment. A control unit is configured to analyze the ambient air pressure and the ambient air temperature in relation to a breakdown voltage for the UV lamp. The control unit is further configured to modify at least one aspect of the UV lamp in response to the ambient air pressure and the ambient air temperature yielding a breakdown threshold that is less than the breakdown voltage.

In at least one example, the environment is an internal cabin of a vehicle.

In at least one example, the control unit is in communication with the power source. The the at least one aspect of the UV lamp includes power supplied to the UV lamp from the power source.

In at least one example, a blower is coupled to the UV lamp. The control unit is in communication with the blower. The blower is configured to blow cooling air into the UV lamp. The at least one aspect of the UV lamp includes air pressure within the UV lamp. The control unit is configured to adaptively control the blower to vary the air pressure within the UV lamp.

In at least one example, the UV lamp includes a housing having an air inlet and an air outlet. The blower is coupled to the air inlet. The air inlet may be larger than the air outlet.

In at least one example, one or more actuators are coupled to the electrodes. The one or more actuators are configured to adjust the spacing between the electrodes. The control unit is in communication with the one or more actuators. The at least one aspect includes the spacing between the electrodes.

In at least one example, the UV lamp includes one or more of the pressure sensor, the temperature sensor, or the control unit. For example, one or both of the pressure sensor or the temperature sensor are secured to a housing of the UV lamp.

Certain examples of the present disclosure provide a method including analyzing, by a control unit, an ambient air pressure as detected by a pressure sensor and an ambient air temperature as detected by a temperature sensor in relation to a breakdown voltage of an ultraviolet (UV) lamp including one or more UV light emitters coupled to electrodes, wherein the UV light emitters are configured to emit UV light within an environment; and modifying, by the control unit, at least one aspect of the UV lamp in response to the ambient air pressure and the ambient air temperature yielding a breakdown threshold that is less than the breakdown voltage.

BRIEF DESCRIPTION OF THE DRAWINGS

Figure 1 illustrates a schematic block diagram of a sanitizing system within an environment.

Figure 2 illustrates a schematic block diagram of a control unit in communication with an ultraviolet (UV) lamp.

Figure 3 illustrates a flow chart of a method of reducing a potential of arcing of within a UV lamp.

Figure 4 illustrates a perspective view of a first side of a UV lamp.

Figure 5 illustrates a perspective view of a second side of the UV lamp.

Figure 6 illustrates a perspective view of the first side of the UV lamp coupled to a blower.

Figure 7 illustrates a perspective view of the second of the UV lamp coupled to the blower.

Figure 8 illustrates a perspective front view of an aircraft.

Figure 9A illustrates a top plan view of an internal cabin of an aircraft.

Figure 9B illustrates a top plan view of an internal cabin of an aircraft.

Figure 10 illustrates a perspective interior view of an internal cabin of an aircraft.

DETAILED DESCRIPTION OF THE DISCLOSURE

The foregoing summary, as well as the following detailed description of certain examples will be better understood when read in conjunction with the appended drawings. As used herein, an element or step recited in the singular and preceded by the word "a" or "an" should be understood as not necessarily excluding the plural of the elements or steps. Further, references to "one example" are not intended to be interpreted as excluding the existence of additional examples that also incorporate the recited features. Moreover, unless explicitly stated to the contrary, examples "comprising" or "having" an element or a plurality of elements having a particular condition can include additional elements not having that condition.

As described herein, examples of the present disclosure provide systems and methods for controlling an ultraviolet lamp to reduce a potential of arcing. In at least one example, the systems and methods are configured to prevent, mitigate, or otherwise reduce arcing of UV light emitters during operation, such as when used to sanitize components within an environment. The systems and methods are configured to control voltage applied to the UV light emitters based on temperature and pressure within the environment, such as in cases where it is required to over-power the UV light emitters to increase irradiance, such as during a flight of an aircraft.

In at least one example, the systems and methods include a power source, a control unit, a UV lamp having electrodes coupled to one or more UV light emitters, a pressure sensor, and a temperature sensor. The control unit is configured to control voltage applied to the UV lamp based on detected temperature and pressure (for example, as the aircraft changes altitude) to reduce a potential of the arcing within the UV lamp (such as between the electrodes). The temperature and pressure data can be known and/or received from a system (such as within a vehicle), or can be generated locally at the apparatus. The control unit can operate as a failsafe, as it can be configured to deactivate the UV lamp if pressure data is lost. In one example, a blower can be included to cool the apparatus to operate at higher altitudes at higher power. In an at least one example, mechanical actuators (such as motors, bellows, or the like) can be controlled to vary the spacing between the electrodes in response to changes in temperature and pressure (for example, altitude changes).

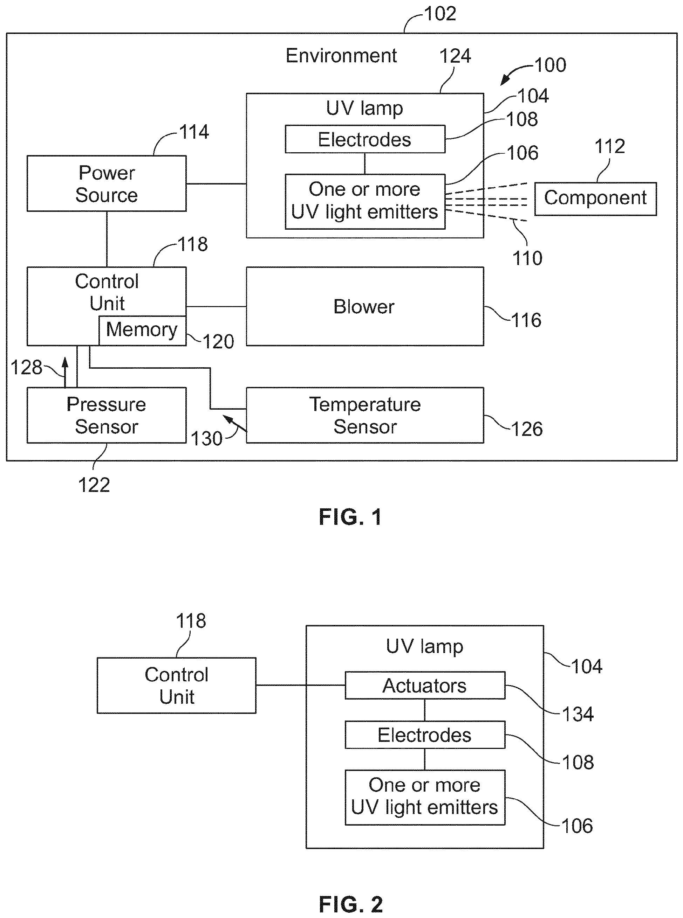

Figure 1 illustrates a schematic block diagram of a sanitizing system 100 within an environment 102, according to an example of the present disclosure. In at least one example, the environment 102 is an internal cabin of a vehicle, such as a commercial aircraft. The air pressure and temperature within the environment 102 may change. For example, as an aircraft ascends, the air pressure and temperature at different altitudes may differ.

The sanitizing system 100 includes an ultraviolet (UV) lamp 104 including one or more UV light emitters 106 electrically coupled to electrodes 108. The UV lamp 104 can be a fixed structure within the environment 102. As another example, the UV lamp 104 can be a mobile and/or portable structure within the environment 102. For example, the UV lamp 104 can be part of a wand assembly. The wand assembly can be coupled to a backpack assembly, a case assembly, a cart assembly, or the like. As another example, the wand assembly can be a standalone unit that is not coupled to another assembly, such as a backpack assembly.

The UV light emitters 106 can be one or more bulb(s), light emitting diode(s) (LEDs), and/or the like. The UV light emitters 106 are configured to emit UV light 110 onto one or more components 112 within the environment to disinfect or otherwise sanitize the components 112. Examples of the components 112 include a floor, a ceiling, a wall, a seat, a countertop, a handle, a faucet, a door, a toilet, and/or the like.

The UV lamp 104 is coupled to a power source 114, such as through one or more wired connections. The power source 114 can be a main power source within the environment, such as a source of alternating current (AC) power. As another example, the power source 114 may be part of the UV lamp 104 itself. That is, the UV lamp 104 can include the power source 114. As another example, the power source 114 can be one or more batteries.

In at least one example, the UV lamp 104 is also coupled to a blower 116, such as through one or more wired or wireless connections. The blower 116 can be a fan, for example. The blower 116 can also be coupled to the power source 114, which provides operational power to the blower 116. The blower 116 is configured to provide cooling air to the UV lamp 104.

A control unit 118 is in communication with the power source 114 and the blower 116, such as through one or more wired or wireless connections. Optionally, the control unit 118 is in communication with only one of the power source 114 or the blower 116. In at least one example, the control unit 118 is separate and remote from the UV lamp 104. In at least one other example, the UV lamp 104 includes the control unit 118.

The control unit 118 is coupled to or otherwise includes a memory 120. The memory 120 stores breakdown voltage data for the UV lamp 104. The breakdown voltage data includes information regarding the breakdown voltage (that is, the voltage applied to the electrodes 108 that causes arcing) for the UV lamp 104 at various altitudes, air pressures, air temperatures, and the like. For example, the breakdown voltage data includes information regarding the breakdown voltage for the UV lamp 104 as the one or more UV light emitters 106 emit the UV light 110 at sea level to 100,000 feet (or more) above sea level.

The control unit 118 is also in communication with a pressure sensor 122 within the environment 102, such as through one or more wired or wireless connections. The pressure sensor 122 is configured to detect the ambient pressure within the environment 102 that surrounds the UV lamp 104. As an example, the pressure sensor 122 can be an electronic barometer. In at least one example, the pressure sensor 122 is separate and distinct from the UV lamp 104. As another example, the pressure sensor 122 is mounted to an external portion of the UV lamp 104, such as an external surface of a housing 124 of the UV lamp 104. Optionally, the control unit 118 may not be in communication with the pressure sensor 122. Instead, the control unit 118 can be in communication with another component, such as an onboard computer of a vehicle, that receives pressure data, such as from the pressure sensor 122.

The control unit 118 is also in communication with a temperature sensor 126 within the environment 102, such as through one or more wired or wireless connections. The temperature sensor 126 is configured to detect the ambient temperature within the environment 102 that surrounds the UV lamp 104. As an example, the temperature sensor 126 can be an electronic thermometer or thermostat. In at least one example, the temperature sensor 126 is separate and distinct from the UV lamp 104. As another example, the temperature sensor 126 is mounted to an external portion of the UV lamp 104, such as an external surface of the housing 124 of the UV lamp 104. Optionally, the control unit 118 may not be in communication with the temperature sensor 126. Instead, the control unit 118 can be in communication with another component, such as an onboard computer of a vehicle, that receives temperature data, such as from the pressure sensor 126.

The ambient pressure and ambient temperature can vary. For example, when the environment 102 is an internal cabin of a vehicle, such as a commercial aircraft, the ambient pressure and ambient temperature change as the altitude above sea level of the vehicle changes.

A voltage value, such as a breakdown voltage or a breakdown threshold, is related to ambient air pressure and ambient air temperature. When ambient air pressure and ambient air temperature are known, the breakdown voltage and the breakdown threshold can be determined. As such, ambient air pressure and ambient air temperature can yield such voltage values.

The breakdown voltage is the voltage necessary to start a discharge or electric arc between two electrodes in a gas a function or pressure and distance between the electrodes, as set forth in Paschen's law. In particular,

$$V_B = B_{pd}/\ln(A_{pd}) - \ln\left[\ln(1 + 1/\gamma_{se})\right]$$

where $V_B$ is the breakdown voltage in volts, p is the pressure in pascals, d is the gap distance in meters, $\gamma_{se}$ is the secondary electron emission coefficient, A is the saturation ionization in the gas at a part E/p (electric field/pressure), and B is related to the excitation and ionization energies. For at air standard conditions for temperature and pressure, the voltage needed to arc a 1 meter gap is about 3.4 MV.

In a least one example, data regarding ambient pressure and ambient temperature can be used to control voltage from the power supply, such as via a lookup table. The lookup table can be stored in the memory coupled to the control unit 118.

In operation, the control unit 118 receives a pressure signal 128 output by the pressure sensor 122. The pressure signal 128 is indicative of the ambient air pressure of the environment 102 that surrounds the UV lamp 104. The control unit 118 also receives a temperature signal 130 output by the temperature sensor 126. The temperature signal 130 is indicative of the ambient air temperature of the environment 102 that surrounds the UV lamp 104.

The control unit 118 receives the pressure signal 128 from the pressure sensor 122 and the temperature signal 130 from the temperature sensor 126. The control unit 118 analyzes the pressure signal 128 (for example, air pressure data regarding, such as indicative of, the ambient air pressure) and the temperature signal 130 (for example, air temperature data regarding, such as indicative of, the ambient air temperature) to determine the ambient air pressure and the ambient air temperature, respectively, of the environment surrounding the UV lamp 104. The control unit 118 analyzes (for example, compares) the ambient air pressure (for example, the data regarding the ambient air pressure) and the ambient air temperature (for example, the data regarding the ambient air temperature) with the breakdown voltage data, which is stored in the memory 120, for the UV lamp 104. If the ambient air pressure and the ambient air temperature are such that the power currently supplied to the UV lamp 104 by the power source 114 is currently below the breakdown voltage, the control unit 118 allows the UV lamp 104 to continue operating in a normal fashion (that is, at the power level provided by the power source 114).

If, however, the ambient air pressure and the ambient air temperature are such that the voltage applied to the UV lamp 104 by the power source 114 is such that a breakdown threshold is reached, the control unit 118 reduces the power provided by the power source 114 (for example, by reducing the voltage applied to the UV lamp 104). The breakdown threshold is a voltage value based on the ambient air pressure and the ambient air temperature. The ambient air pressure and the ambient air temperature yield a voltage value for the UV lamp 104. The voltage value is compared with the breakdown threshold, which is itself a voltage value. The breakdown voltage can be a predetermined percentage of the breakdown voltage. For example, the breakdown threshold can be between 90-99% of the breakdown voltage. As such, when the breakdown threshold is met, the control unit 118 reduces the power supplied by the power source 114 (such as a 10-20% reduction in power) to ensure that the breakdown voltage does not occur. Optionally, the breakdown threshold can be more than 99%, but less than 100% of the breakdown voltage. As another example, the breakdown threshold can be less than 90% of the breakdown voltage, such as 75% of the breakdown voltage. Also, the reduction in power supplied to the UV lamp 104 can be less than a 10% reduction in power, or more than a 20% reduction in power. As another example, the breakdown threshold can be in terms other than a percentage of the breakdown voltage. For example, the breakdown threshold can be 1-5 V less than the breakdown voltage.

As an example, the maximum power for the UV lamp 104 at an 8000 foot pressure altitude may be approximately 54 W. The control unit 118 is configured to control the power supplied to the UV lamp 104 at such air pressure to less than 54 W, thereby ensuring that the breakdown voltage for the UV lamp 104 does not occur.

In at least one example, in addition to, or in place of, controlling the power applied to the UV lamp 104, the control unit 118 is configured to adaptively control the blower 116. The blower 116 operates to cool the UV lamp 104. If, however, the ambient air pressure and the ambient air temperature are such that the voltage applied to the UV lamp 104 by the power source 114 is such that a breakdown threshold is reached, the control unit 118 activates the blower 116, and/or increases the power supplied to the blower 116 (so as to increase fan speed, for example). By activating and/or increasing the power supplied to the blower 116, the blower 116 supplies additional cooling air within the UV lamp 104. The additional cooling air increases the air pressure within the UV lamp 104. The increased air pressure increases the breakdown voltage. As such, instead of (or in addition to) reducing the power supplied to the UV lamp 104 in response to the breakdown threshold being reached, the control unit 118 can operate the blower 116 to increase the air pressure within the UV lamp 104, which allows the UV lamp 104 to be operated at an increased power without the breakdown voltage being reached.

As described herein, the control unit 118 is configured to modify at least one aspect of the UV lamp 104 in response to the ambient pressure and the ambient temperature yielding the breakdown threshold. For example, the aspect is power supplied to the UV lamp 104. As another example, the aspect is air pressure within the UV lamp 104. As another example, the at least one aspect is a first aspect, such as power supplied to the UV lamp 104, and a second aspect, such as air pressure within the UV lamp 104.

As described herein, the control unit 118 is configured to control the power applied to the UV lamp 104 from the power source 114 in response to the detected air pressure and the air temperature within the environment 102 to prevent, mitigate, or otherwise reduce the potential of arcing within the UV lamp 104. As another example, the control unit 118 is configured to control the blower 116 (such as to increase the air pressure within the UV lamp 104) in response to the detected air pressure and the air temperature within the environment 102 to prevent, mitigate, or otherwise reduce the potential of arcing within the UV lamp 104. As another example, the control unit 118 is configured to (a) control the power applied to the UV lamp 104 from the power source 114, and (b) control the blower 116 in response to the detected air pressure and the air temperature within the environment 102 to prevent, mitigate, or otherwise reduce the potential of arcing within the UV lamp 104.

Figure 2 illustrates a schematic block diagram of the control unit 118 in communication with the UV lamp 104, according to an example of the present disclosure. In at least one example, the control unit 118 is in communication with one or more actuators 134 of the UV lamp 104. The actuator(s) 134 can be or include one or more motor(s), bellow(s), track(s), arm(s), and/or the like that are coupled to the electrodes 108. The actuators 134 are configured to move the electrodes 108 relative to one another. In at least one example, an aspect of the UV lamp 104 that can be modified by the control unit 118 in response to the breakdown threshold being met is that the UV lamp 104 can move the electrodes 108 to ensure that the breakdown voltage does not occur. For example, the control unit 118 can operate the actuators 134 to move the electrodes further apart to reduce the potential of the breakdown voltage occurring.

The example shown in Figure 2 can be used in conjunction with the examples described with respect to Figure 1. In at least one example, the control unit 118 can operate the actuators 134 to control a spacing between the electrodes in response to the breakdown threshold being reached instead of reducing the power supplied to the UV lamp 104, or controlling the blower 116. In at least one example, the control unit 118 is not coupled to actuators of a UV lamp. That is, in at least one example, the control unit 118 does not operate to move the electrodes 108 in response to the breakdown threshold being reached. For example, the UV lamp 104 may not include the actuators.

Referring to Figures 1 and 2, the system 100 includes the UV lamp 104 including one or more UV light emitters 106 coupled to the electrodes 108. The UV light emitters 106 are configured to emit the UV light 110 within the environment 102. The power source 114 is coupled to the UV lamp 104. The power source 114 is configured to supply power to the UV lamp 104. The pressure sensor 122 is configured to detect an ambient air pressure within the environment 102. The temperature sensor 126 is configured to detect an ambient air temperature within the environment 102. The control unit 118 is in communication with the pressure sensor 122 and the temperature sensor 126. The control unit 118 is configured to analyze the ambient air pressure and the ambient air temperature in relation to a breakdown voltage for the UV lamp 104 (that is, the voltage at which arcing occurs between the electrodes 108 at a current (a) spacing therebetween, (b) ambient air pressure, and (c) ambient temperature). The control unit 118 is further configured to modify at least one aspect of the UV lamp 104 in response to the ambient air pressure and the ambient air temperature yielding a breakdown threshold that is less than the breakdown voltage.

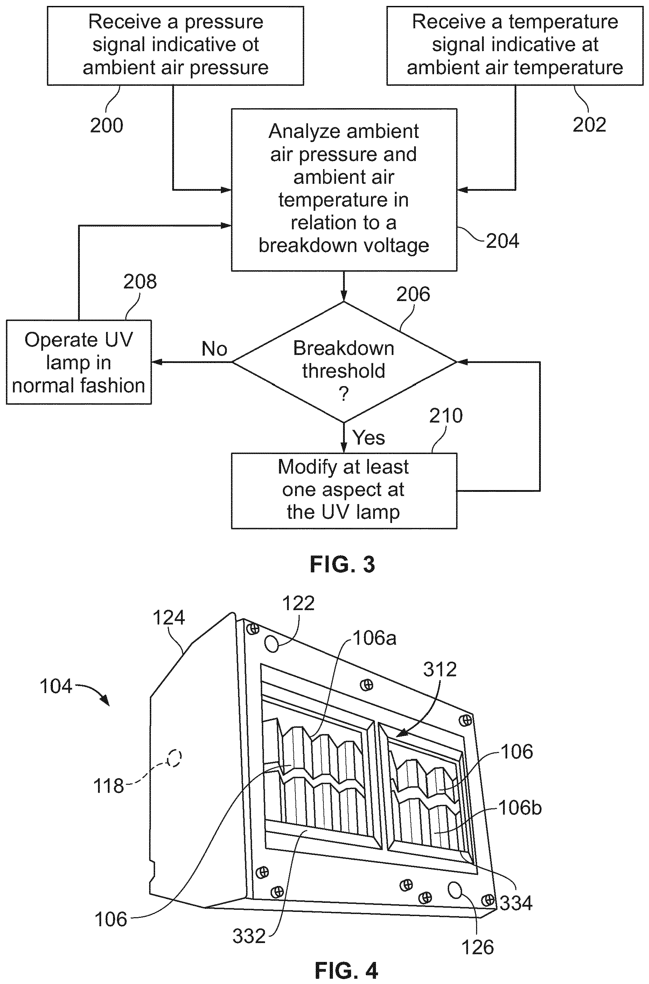

Figure 3 illustrates a flow chart of a method of reducing a potential of arcing of within a UV lamp, according to an example of the present disclosure. Referring to Figures 1-3, at 200, the control unit 118 receives the pressure signal 128 indicative of ambient air pressure within the environment 102 from the pressure sensor 122. At 202, the control unit 118 receives the temperature signal 130 indicative of ambient air temperature within the environment 102 from the temperature sensor 126. Steps 200 and 202 can occur simultaneously. Optionally, step 200 can occur before 202, or vice versa.

At 204, the control unit 118 analyzes the ambient air pressure and the ambient air pressure in relation to a breakdown voltage for the UV lamp 104. At 206, the control unit 118 determines if the breakdown threshold (which is less than the breakdown voltage) has been met. If not, the method proceeds from 206 to 208, at which the UV lamp 104 is (or can be) operated in a normal fashion. The method then returns to 204.

If, however, the breakdown threshold has been met at 206, the control unit 118 modifies at least one aspect of the UV lamp 104 at 210. For example, the at least one aspect includes one or more of power supplied to the UV lamp 104, air pressure within the UV lamp 104 (as controlled through operation of the blower 116), and/or a distance or spacing between the electrodes 108 of the UV lamp 104 (as controlled through the one or more actuators 134).

As used herein, the term "control unit," "central processing unit," "unit," "CPU," "computer," or the like can include any processor-based or microprocessor-based system including systems using microcontrollers, reduced instruction set computers (RISC), application specific integrated circuits (ASICs), logic circuits, and any other circuit or processor including hardware, software, or a combination thereof capable of executing the functions described herein. Such are exemplary only, and are thus not intended to limit in any way the definition and/or meaning of such terms. For example, the control unit 118 can be or include one or more processors that are configured to control operation thereof, as described herein.

The control unit 118 is configured to execute a set of instructions that are stored in one or more data storage units or elements (such as one or more memories), in order to process data. For example, the control unit 118 can include or be coupled to one or more memories. The data storage units can also store data or other information as desired or needed. The data storage units can be in the form of an information source or a physical memory element within a processing machine. The one or more data storage units or elements can comprise volatile memory or nonvolatile memory, or can include both volatile and nonvolatile memory. As an example, the nonvolatile memory can comprise read only memory (ROM), programmable ROM (PROM), electrically programmable ROM (EPROM), electrically erasable PROM (EEPROM), and/or flash memory and volatile memory can include random access memory (RAM), which can act as external cache memory. The data stores of the disclosed systems and methods is intended to comprise, without being limited to, these and any other suitable types of memory.

The set of instructions can include various commands that instruct the control unit 118 as a processing machine to perform specific operations such as the methods and processes of the various examples of the subject matter described herein. The set of instructions can be in the form of a software program. The software can be in various forms such as system software or application software. Further, the software can be in the form of a collection of separate programs, a program subset within a larger program or a portion of a program. The software can also include modular programming in the form of object-oriented programming. The processing of input data by the processing machine can be in response to user commands, or in response to results of previous processing, or in response to a request made by another processing machine.

The diagrams of examples herein illustrate one or more control or processing units, such as the control unit 118. It is to be understood that the processing or control units can represent circuits, circuitry, or portions thereof that can be implemented as hardware with associated instructions (e.g., software stored on a tangible and non-transitory computer readable storage medium, such as a computer hard drive, ROM, RAM, or the like) that perform the operations described herein. The hardware can include state machine circuitry hardwired to perform the functions described herein. Optionally, the hardware can include electronic circuits that include and/or are connected to one or more logic-based devices, such as microprocessors, processors, controllers, or the like. Optionally, the control unit 118 can represent processing circuitry such as one or more of a field programmable gate array (FPGA), application specific integrated circuit (ASIC), microprocessor(s), and/or the like. The circuits in various examples can be configured to execute one or more algorithms to perform functions described herein. The one or more algorithms can include aspects of examples disclosed herein, whether or not expressly identified in a flowchart or a method.

As used herein, the terms "software" and "firmware" are interchangeable, and include any computer program stored in a data storage unit (for example, one or more memories) for execution by a computer, including RAM memory, ROM memory, EPROM memory, EEPROM memory, and non-volatile RAM (NVRAM) memory. The above data storage unit types are exemplary only, and are thus not limiting as to the types of memory usable for storage of a computer program.

Figure 4 illustrates a perspective view of a first side (such as a bottom or top) of the UV lamp 104, according to an example of the present disclosure. The UV lamp 104 includes the housing 124 that retains a plurality of UV light emitters 106 that are configured to emit UV light through an aperture 312. As shown, the UV lamp 104 includes a first plurality of UV light emitters 106a and a second plurality of UV light emitters 106b. The first plurality of UV light emitters 106a are contained within a first sub-housing 332, and the second plurality of UV light emitters 106b are contained within a second sub-housing 334 that is distinct from the first sub-housing 332. Each of the first sub-housing 332 and the second sub-housing 334 can contain more or less UV light emitters 106 than shown. Optionally, the UV lamp 104 can include a single sub-housing that retains all of the UV light emitters 106. In at least one example, the UV lamp 104 can include a single UV light emitter 106, instead a plurality of UV light emitters 106. The UV lamp 104 shown in Figure 4 is merely an example. The UV lamp 104 can be sized and shaped differently than shown in Figure 4.

In at least one example, the pressure sensor 122 is secured to the housing 124. As such, the UV lamp 104 can include the pressure sensor 122. In at least one other example, the pressure sensor 122 is separate and distinct from the UV lamp 104.

In at least one example, the temperature sensor 126 is secured to the housing 124. Accordingly, the UV lamp 104 can include the temperature sensor 126. In at least one other example, the temperature sensor 126 is separate and distinct from the UV lamp 104.

In at least one example, the UV lamp 104 includes the control unit 118. For example, the control unit 118 can contained within the housing 124. In at least one other example, the control unit 118 is separate and distinct from the UV lamp 104.

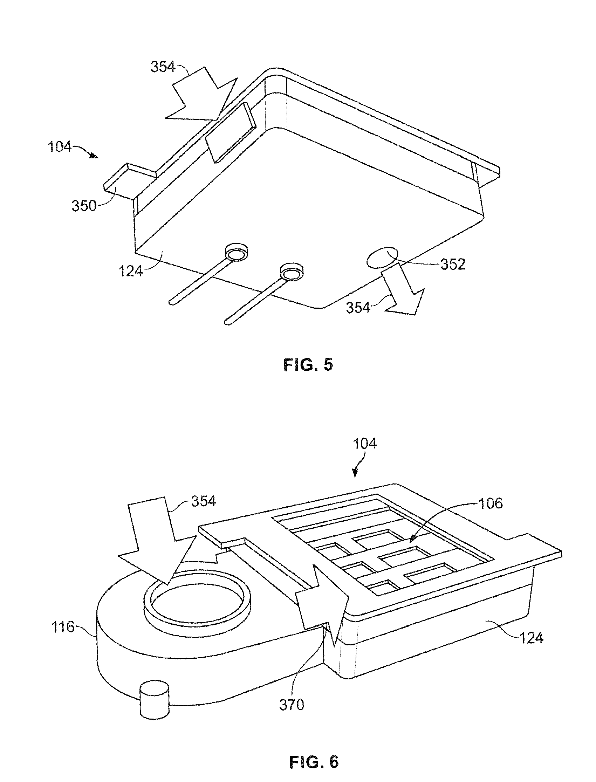

Figure 5 illustrates a perspective view of a second side (opposite from the first side) of the UV lamp 104, according to an example of the present disclosure. The housing 124 includes an air inlet 350, and at least one air outlet 352. Air 354 is drawn into the housing 124, so as to cool the electrodes 108 and UV light emitters 106 (shown in Figure 1, for example), and passes out through the air outlet 352. As shown, the air inlet 350 can be larger than the air outlet 352. For example, the air inlet 350 is at least twice the size of the air outlet 352. The air 354 can be drawn into the housing 124 through the larger air inlet 350 faster than the air 354 can escape through the smaller air outlet 352, thereby increasing the air pressure within the UV lamp 104.

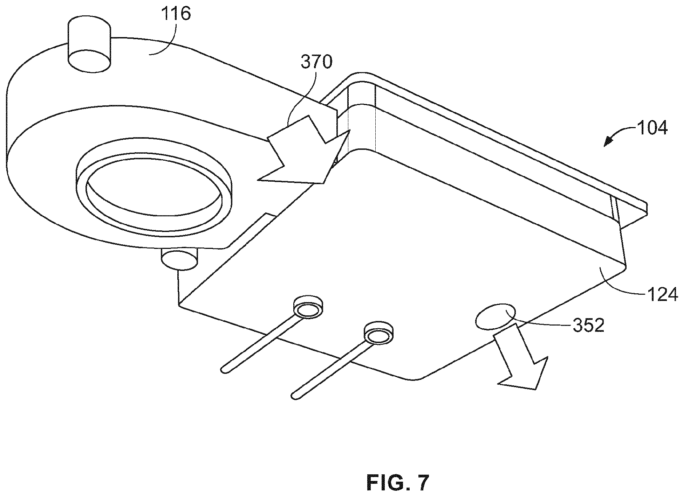

Figure 6 illustrates a perspective view of the first side of the UV lamp 104 coupled to the blower 116, according to an example of the present disclosure. Figure 7 illustrates a perspective view of the second of the UV lamp 104 coupled to the blower 116. Referring to Figures 5-7, an outlet 370 of the blower 116 is connected to the air inlet 350. As described with respect to Figure 1, the control unit 118 is configured to control the blower 116 to increase the air pressure within the UV lamp 104, such as by increasing a fan speed of the blower 116 to draw in more air 354 into the housing 124 at a faster rate than the air 354 can pass out of the air outlet 352.

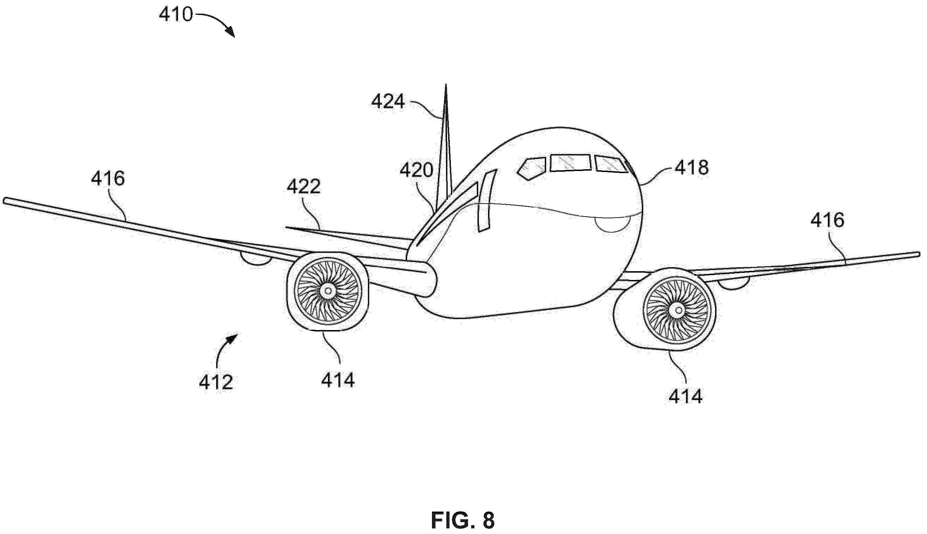

Figure 8 illustrates a perspective front view of an aircraft 410, according to an example of the present disclosure. The aircraft 410 includes a propulsion system 412 that includes engines 414, for example. Optionally, the propulsion system 412 may include more engines 414 than shown. The engines 414 are carried by wings 416 of the aircraft 410. In other examples, the engines 414 may be carried by a fuselage 418 and/or an empennage 420. The empennage 420 may also support horizontal stabilizers 422 and a vertical stabilizer 424.

The fuselage 418 of the aircraft 410 defines an internal cabin 430, which includes a flight deck or cockpit, one or more work sections (for example, galleys, personnel carry-on baggage areas, and the like), one or more passenger sections (for example, first class, business class, and coach sections), one or more lavatories, and/or the like. The internal cabin 430 includes one or more lavatory systems, lavatory units, or lavatories, as described herein. The internal cabin 430 is an example of the environment 102, shown in Figure 1.

Examples of the present disclosure are used within the internal cabin 430. Alternatively, instead of an aircraft, examples of the present disclosure may be used with various other vehicles, such as automobiles, buses, locomotives and train cars, watercraft, and the like. Further, examples of the present disclosure may be used with respect to fixed structures, such as commercial and residential buildings.

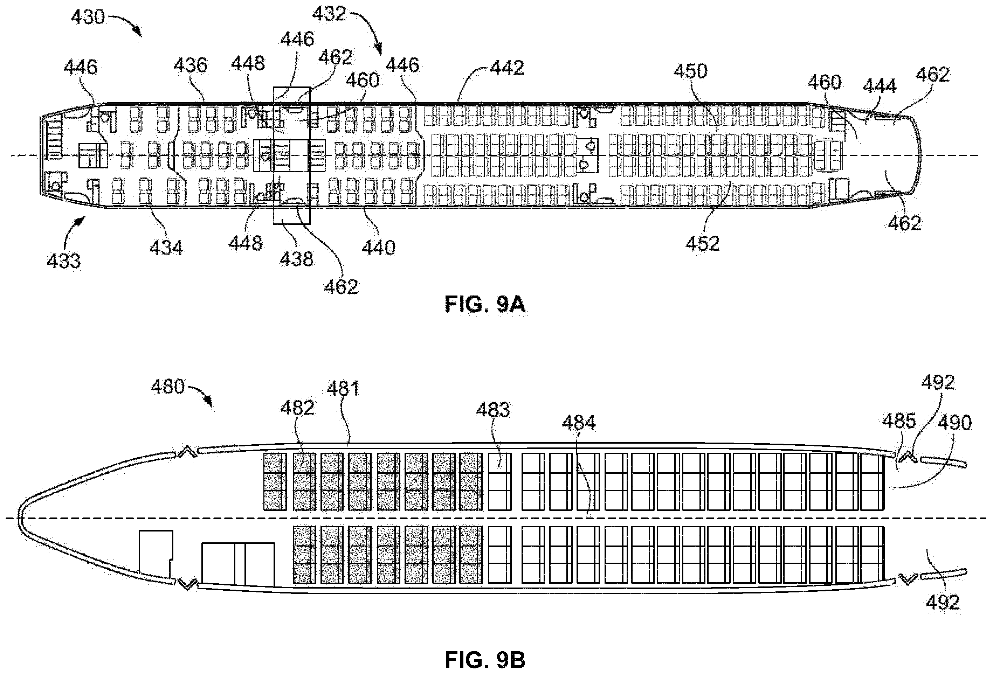

Figure 9A illustrates a top plan view of an internal cabin 430 of an aircraft, according to an example of the present disclosure. The internal cabin 430 may be within the fuselage 432 of the aircraft, such as the fuselage 418 of Figure 8. For example, one or more fuselage walls may define the internal cabin 430. The internal cabin 430 includes multiple sections, including a front section 433, a first class section 434, a business class section 436, a front galley station 438, an expanded economy or coach section 440, a standard economy of coach section 442, and an aft section 444, which may include multiple lavatories and galley stations. It is to be understood that the internal cabin 430 may include more or less sections than shown. For example, the internal cabin 430 may not include a first class section, and may include more or less galley stations than shown. Each of the sections may be separated by a cabin transition area 446, which may include class divider assemblies between aisles 448.

As shown in Figure 9A, the internal cabin 430 includes two aisles 450 and 452 that lead to the aft section 444. Optionally, the internal cabin 430 may have less or more aisles than shown. For example, the internal cabin 430 may include a single aisle that extends through the center of the internal cabin 430 that leads to the aft section 444.

The aisles 448, 450, and 452 extend to egress paths or door passageways 460. Exit doors 462 are located at ends of the egress paths 460. The egress paths 460 may be perpendicular to the aisles 448, 450, and 452. The internal cabin 430 may include more egress paths 460 at different locations than shown. Examples of the present disclosure shown and described with respect to Figures 1-7 may be used within the internal cabin 430.

Figure 9B illustrates a top plan view of an internal cabin 480 of an aircraft, according to an example of the present disclosure. The internal cabin 480 is an example of the internal cabin 430 shown in Figure 8. The internal cabin 480 may be within a fuselage 481 of the aircraft. For example, one or more fuselage walls may define the internal cabin 480. The internal cabin 480 includes multiple sections, including a main cabin 482 having passenger seats 483, and an aft section 485 behind the main cabin 482. It is to be understood that the internal cabin 480 may include more or less sections than shown.

The internal cabin 480 may include a single aisle 484 that leads to the aft section 485. The single aisle 484 may extend through the center of the internal cabin 480 that leads to the aft section 485. For example, the single aisle 484 may be coaxially aligned with a central longitudinal plane of the internal cabin 480.

The aisle 484 extends to an egress path or door passageway 490. Exit doors 492 are located at ends of the egress path 490. The egress path 490 may be perpendicular to the aisle 484. The internal cabin 480 may include more egress paths than shown. Examples of the present disclosure shown and described with respect to Figures 1-7 may be used within the internal cabin 480.

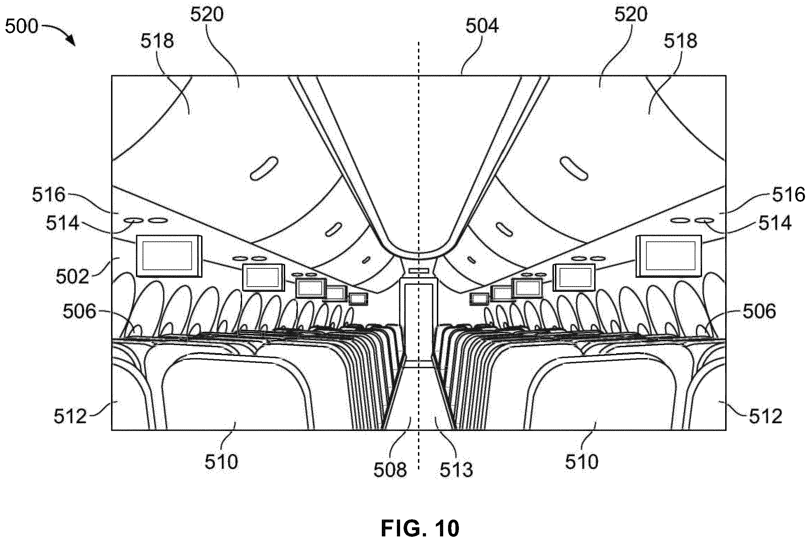

Figure 10 illustrates a perspective interior view of an internal cabin 500 of an aircraft, according to an example of the present disclosure. The internal cabin 500 includes outboard walls 502 connected to a ceiling 504. Windows 506 may be formed within the outboard walls 502. A floor 508 supports rows of seats 510. As shown in Figure 10, a row 512 may include two seats 510 on either side of an aisle 513. However, the row 512 may include more or less seats 510 than shown. Additionally, the internal cabin 500 may include more aisles than shown.

Passenger service units (PSUs) 514 are secured between an outboard wall 502 and the ceiling 504 on either side of the aisle 513. The PSUs 514 extend between a front end and rear end of the internal cabin 500. For example, a PSU 514 may be positioned over each seat 510 within a row 512. Each PSU 514 may include a housing 516 that generally contains vents, reading lights, an oxygen bag drop panel, an attendant request button, and other such controls over each seat 510 (or groups of seats) within a row 512.

Overhead stowage bin assemblies 518 are secured to the ceiling 504 and/or the outboard wall 502 above and inboard from the PSU 514 on either side of the aisle 513. The overhead stowage bin assemblies 518 are secured over the seats 510. The overhead stowage bin assemblies 518 extend between the front and rear end of the internal cabin 500. Each stowage bin assembly 518 may include a pivot bin or bucket 520 pivotally secured to a strongback (hidden from view in Figure 10). The overhead stowage bin assemblies 518 may be positioned above and inboard from lower surfaces of the PSUs 514. The overhead stowage bin assemblies 518 are configured to be pivoted open in order to receive passenger carry-on baggage and personal items, for example.

Examples of the present disclosure shown and described with respect to Figures 1-7 can be used in the internal cabin 500. The internal cabin 500 is an example of the environment 102 shown in Figure 1.

Further, the disclosure comprises examples according to the following clauses:

Clause 1. A system, comprising:

an ultraviolet (UV) lamp including one or more UV light emitters coupled to electrodes, wherein the UV light emitters are configured to emit UV light within an environment;

a power source coupled to the UV lamp, wherein the power source is configured to supply power to the UV lamp;

a pressure sensor configured to detect an ambient air pressure within the environment;

a temperature sensor configured to detect an ambient air temperature within the environment; and

a control unit configured to analyze air pressure data regarding the ambient air pressure and air temperature data regarding the ambient air temperature in relation to a breakdown voltage for the UV lamp, and wherein the control unit is further configured to modify at least one aspect of the UV lamp in response to the ambient air pressure and the ambient air temperature yielding a breakdown threshold that is less than the breakdown voltage.

Clause 2. The system of Clause 1, wherein the environment is an internal cabin of a vehicle.

Clause 3. The system of Clauses 1 or 2, wherein the control unit is in communication with the power source, and wherein the at least one aspect of the UV lamp comprises power supplied to the UV lamp from the power source.

Clause 4. The system of any of Clauses 1-3, further comprising a blower coupled to the UV lamp, wherein the control unit is in communication with the blower, wherein the blower is configured to blow cooling air into the UV lamp, wherein the at least one aspect of the UV lamp comprises air pressure within the UV lamp, and wherein the control unit is configured to adaptively control the blower to vary the air pressure within the UV lamp.

Clause 5. The system of Clause **4,** wherein the UV lamp comprises a housing having an air inlet and an air outlet, wherein the blower is coupled to the air inlet.

Clause 6. The system of Clause 5, wherein the air inlet is larger than the air outlet.

Clause 7. The system of any of Clauses 1-6, further comprising one or more actuators coupled to the electrodes, wherein the one or more actuators are configured to adjust the spacing between the electrodes, wherein the control unit is in communication with the one or more actuators, and wherein the at least one aspect includes the spacing between the electrodes.

Clause 8. The system of any of Clauses 1-7, wherein the UV lamp includes one or more of the pressure sensor, the temperature sensor, or the control unit.

Clause 9. The system of Clause 8, wherein one or both of the pressure sensor or the temperature sensor are secured to a housing of the UV lamp.

Clause 10. A method, comprising:

analyzing, by a control unit, an ambient air pressure as detected by a pressure sensor and an ambient air temperature as detected by a temperature sensor in relation to a breakdown voltage of an ultraviolet (UV) lamp including one or more UV light emitters coupled to electrodes, wherein the UV light emitters are configured to emit UV light within an environment; and

modifying, by the control unit, at least one aspect of the UV lamp in response to the ambient air pressure and the ambient air temperature yielding a breakdown threshold that is less than the breakdown voltage.

Clause 11. The method of Clause 10, wherein the environment is an internal cabin of a vehicle.

Clause 12. The method of Clauses 10 or 11, wherein the at least one aspect of the UV lamp comprises power supplied to the UV lamp from the power source.

Clause 13. The method of any of Clauses 10-12, wherein the at least one aspect of the UV lamp comprises air pressure within the UV lamp, and wherein said modifying comprises adaptively controlling the blower to vary the air pressure within the UV lamp.

Clause 14. The method of any of Clauses 10-13, wherein the at least one aspect includes spacing between the electrodes.

Clause 15. The method of any of Clauses 10-14, wherein the UV lamp includes one or more of the pressure sensor, the temperature sensor, or the control unit.

Clause 16. The method of Clause 15, wherein one or both of the pressure sensor or the temperature sensor are secured to a housing of the UV lamp.

Clause 17. A system, comprising:

an ultraviolet (UV) lamp including one or more UV light emitters coupled to electrodes, and actuators coupled to the electrodes, wherein the UV light emitters are configured to emit UV light within an environment, and wherein the actuators are configured to adjust the spacing between the electrodes;

a power source coupled to the UV lamp, wherein the power source is configured to supply power to the UV lamp;

a blower coupled to the UV lamp, wherein the blower is configured to blow cooling air into the UV lamp;

a pressure sensor configured to detect an ambient air pressure within the environment;

a temperature sensor configured to detect an ambient air temperature within the environment; and

a control unit configured to analyze air pressure data regarding the ambient air pressure and air temperature data regarding the ambient air temperature in relation to a breakdown voltage for the UV lamp, and wherein the control unit is further configured to modify aspects of the UV lamp in response to the ambient air pressure and the ambient air temperature yielding a breakdown threshold that is less than the breakdown voltage,

wherein the control unit is in communication with the power source, and wherein the aspects of the UV lamp comprise power supplied to the UV lamp from the power source,

wherein the control unit is in communication with the blower, wherein the aspects of the UV lamp further comprise air pressure within the UV lamp, and wherein the control unit is configured to adaptively control the blower to vary the air pressure within the UV lamp, and

wherein the control unit is in communication with the one or more actuators, and wherein the aspects include the spacing between the electrodes.

Clause 18. The system of Clause 17, wherein the environment is an internal cabin of a vehicle.

Clause 19. The system of Clauses 17 or 18, wherein the UV lamp comprises a housing having an air inlet and an air outlet, wherein the blower is coupled to the air inlet, and herein the air inlet is larger than the air outlet.

Clause 20. The system of any of Clauses 17-19, wherein the pressure sensor and the temperature sensor are secured to a housing of the UV lamp.

As described herein, examples of the present disclosure provide systems and methods for reducing potential arcing of a UV lamp. Further, examples of the present disclosure provide systems and methods for adapting operation of a UV lamp at various altitudes and air pressures.
While various spatial and directional terms, such as top, bottom, lower, mid, lateral, horizontal, vertical, front and the like can be used to describe examples of the present disclosure, it is understood that such terms are merely used with respect to the orientations shown in the drawings. The orientations can be inverted, rotated, or otherwise changed, such that an upper portion is a lower portion, and vice versa, horizontal becomes vertical, and the like.
As used herein, a structure, limitation, or element that is "configured to" perform a task or operation is particularly structurally formed, constructed, or adapted in a manner corresponding to the task or operation. For purposes of clarity and the avoidance of doubt, an object that is merely capable of being modified to perform the task or operation is not "configured to" perform the task or operation as used herein.
It is to be understood that the above description is intended to be illustrative, and not restrictive. For example, the above-described examples (and/or aspects thereof) can be used in combination with each other. In addition, many modifications can be made to adapt a particular situation or material to the teachings of the various examples of the disclosure without departing from their scope. While the dimensions and types of materials described herein are intended to define the parameters of the various examples of the disclosure, the examples are by no means limiting and are exemplary examples. Many other examples will be apparent to those of skill in the art upon reviewing the above description. The scope of the various examples of the disclosure should, therefore, be determined with reference to the appended claims, along with the full scope of equivalents to which such claims are entitled. In the appended claims and the detailed description herein, the terms "including" and "in which" are used as the plain-English equivalents of the respective terms "comprising" and "wherein." Moreover, the terms "first," "second," and "third," etc. are used merely as labels, and are not intended to impose numerical requirements on their objects.
This written description uses examples to disclose the various examples of the disclosure, including the best mode, and also to enable any person skilled in the art to practice the various examples of the disclosure, including making and using any devices or systems and performing any incorporated methods. The patentable scope of the various examples of the disclosure is defined by the claims,

Claims

1. A system (100), comprising:

an ultraviolet (UV) lamp (104) including one or more UV light emitters (106) coupled to electrodes, wherein the UV light emitters (106) are configured to emit UV light (110) within an environment;
a power source (114) coupled to the UV lamp (104), wherein the power source (114) is configured to supply power to the UV lamp (104);

a pressure sensor (122) configured to detect an ambient air pressure within the environment;

a temperature sensor (126) configured to detect an ambient air temperature within the environment; and

a control unit (118) configured to analyze air pressure data regarding the ambient air pressure and air temperature data regarding the ambient air temperature in relation to a breakdown voltage for the UV lamp (104),

wherein the control unit (118) is further configured to modify at least one aspect of the UV lamp (104) in response to the ambient air pressure and the ambient air temperature yielding a breakdown threshold that is less than the breakdown voltage.

2. The system (100) of claim 1 , wherein the environment is an internal cabin of a vehicle.

3. The system (100) of any of the claims 1-2, wherein the control unit (118) is in communication with the power source (114), and wherein the at least one aspect of the UV lamp (104) comprises power supplied to the UV lamp (104) from the power source (114).

4. The system (100) of any of the claims 1-3, further comprising a blower (116) coupled to the UV lamp (104), wherein the control unit (118) is in communication with the blower (116), wherein the blower (116) is configured to blow cooling air into the UV lamp (104), wherein the at least one aspect of the UV lamp (104) comprises air pressure within the UV lamp (104), and wherein the control unit (118) is configured to adaptively control the blower (116) to vary the air pressure within the UV lamp (104).

5. The system (100) of claim 4, wherein the UV lamp (104) comprises a housing (124) having an air inlet and an air outlet, wherein the blower (116) is coupled to the air inlet.

6. The system (100) of claim 5, wherein the air inlet is larger than the air outlet.

7. The system (100) of any of the claims 1-6, further comprising one or more actuators (134) coupled to the electrodes, wherein the one or more actuators (134) are configured to adjust the spacing between the electrodes, wherein the control unit (118) is in communication with the one or more actuators (134), and wherein the at least one aspect includes the spacing between the electrodes.

8. The system (100) of any of the claims 1-7, wherein the UV lamp (104) includes one or more of the pressure sensor (122), the temperature sensor (126), or the control unit (118).

9. The system (100) of claim 8, wherein one of the pressure sensor (122) or the temperature sensor (126) is secured to a housing (124) of the UV lamp (104).

10. The system (100) of claim 8, wherein both the pressure sensor (122) and the temperature sensor (126) are secured to a housing (124) of the UV lamp (104).

11. A method, comprising:

analyzing, by a control unit, an ambient air pressure as detected by a pressure sensor and an ambient air temperature as detected by a temperature sensor in relation to a breakdown voltage of an ultraviolet (UV) lamp including one or more UV light emitters coupled to electrodes, wherein the UV light emitters are configured to emit UV light within an environment; and modifying, by the control unit, at least one aspect of the UV lamp in response to the ambient air pressure and the ambient air temperature yielding a breakdown threshold that is less than the breakdown voltage.

12. The method of claim 11, wherein the environment is an internal cabin of a vehicle.

13. The method of claims 11 or 12, wherein the at least one aspect of the UV lamp comprises power supplied to the UV lamp from the power source.

14. The method of any of the claims 11-13, wherein the at least one aspect of the UV lamp comprises air pressure within the UV lamp, and wherein said modifying comprises adaptively controlling the blower to vary the air pressure within the UV lamp.

15. The method of any of the claims 11-14 with one or more of the following:

wherein the at least one aspect includes spacing between the electrodes;

wherein the UV lamp includes one or more of the pressure sensor, the temperature sensor or the control unit;

wherein one or both of the pressure sensor or the temperature sensor are secured to a housing of the UV lamp.

**Patentansprüche**

1. System (100), umfassend:

eine Ultraviolettlampe (UV-Lampe) (104), die einen oder mehrere UV-Lichtemitter (106) einschließt, die mit Elektroden gekoppelt sind, wobei die UV-Lichtemitter (106) konfiguriert sind, um UV-Licht (110) innerhalb einer Umgebung zu emittieren;

eine Leistungsquelle (114), die mit der UV-Lampe (104) gekoppelt ist, wobei die Leistungsquelle (114) konfiguriert ist, um der UV-Lampe (104) Leistung zuzuführen;

einen Drucksensor (122), der konfiguriert ist, um einen Umgebungsluftdruck innerhalb der Umgebung zu erfassen;

einen Temperatursensor (126), der konfiguriert ist, um eine Umgebungslufttemperatur innerhalb der Umgebung zu erfassen; und

eine Steuereinheit (118), die konfiguriert ist, um Luftdruckdaten bezüglich des Umgebungsluftdrucks und Lufttemperaturdaten bezüglich der Umgebungslufttemperatur in Bezug auf eine Durchbruchspannung für die UV-Lampe (104) zu analysieren,

wobei die Steuereinheit (118) ferner konfiguriert ist, um mindestens einen Aspekt der UV-Lampe (104) als Reaktion auf den Umgebungsluftdruck und die Umgebungslufttemperatur zu modifizieren, wobei sich eine Durchbruchschwelle ergibt, die geringer als die Durchbruchspannung ist.

2. System (100) nach Anspruch 1, wobei die Umgebung eine Innenkabine eines Fahrzeugs ist.

3. System (100) nach einem der Ansprüche 1 bis 2, wobei die Steuereinheit (118) mit der Leistungsquelle (114) in Verbindung steht und wobei der mindestens eine Aspekt der UV-Lampe (104) Leistung umfasst, die der UV-Lampe (104) von der Leistungsquelle (114) zugeführt wird.

4. System (100) nach einem der Ansprüche 1 bis 3, ferner umfassend ein Gebläse (116), das mit der UV-Lampe (104) gekoppelt ist, wobei die Steuereinheit (118) mit dem Gebläse (116) in Verbindung steht, wobei das Gebläse (116) konfiguriert ist, um Kühlluft in die UV-Lampe (104) zu blasen, wobei der mindestens eine Aspekt der UV-Lampe (104) den Luftdruck innerhalb der UV-Lampe (104) umfasst und wobei die Steuereinheit (118) konfiguriert ist, um das Gebläse (116) adaptiv zu steuern, um den Luftdruck innerhalb der UV-Lampe (104) zu variieren.

5. System (100) nach Anspruch 4, wobei die UV-Lampe (104) ein Gehäuse (124) umfasst, das einen Lufteinlass und einen Luftauslass aufweist, wobei das Gebläse (116) mit dem Lufteinlass gekoppelt ist.

6. System (100) nach Anspruch 5, wobei der Lufteinlass größer als der Luftauslass ist.

7. System (100) nach einem der Ansprüche 1 bis 6, ferner umfassend einen oder mehrere Aktuatoren (134), die mit den Elektroden gekoppelt sind, wobei der eine oder die mehreren Aktuatoren (134) konfiguriert sind, um den Abstand zwischen den Elektroden einzustellen, wobei die Steuereinheit (118) mit dem einen oder den mehreren Aktuatoren (134) in Verbindung steht und wobei der mindestens eine Aspekt den Abstand zwischen den Elektroden umfasst.

8. System (100) nach einem der Ansprüche 1 bis 7, wobei die UV-Lampe (104) eines oder mehrere von dem Drucksensor (122), dem Temperatursensor (126) oder der Steuereinheit (118) umfasst.

9. System (100) nach Anspruch 8, wobei einer des Drucksensors (122) oder des Temperatursensors (126) an einem Gehäuse (124) der UV-Lampe (104) befestigt ist.

10. System (100) nach Anspruch 8, wobei sowohl der Drucksensor (122) als auch der Temperatursensor (126) an einem Gehäuse (124) der UV-Lampe (104) befestigt sind.

11. Verfahren, umfassend:

Analysieren, durch eine Steuereinheit, eines Umgebungsluftdrucks, wie durch einen Drucksensor erfasst, und einer Umgebungslufttemperatur, wie durch einen Temperatursensor erfasst, in Bezug auf eine Durchbruchspannung einer Ultraviolettlampe (UV-Lampe), die einen oder mehrere UV-Lichtemitter einschließt, die mit Elektroden gekoppelt sind, wobei die UV-Lichtemitter konfiguriert sind, um UV-Licht innerhalb einer Umgebung zu emittieren; und

Modifizieren, durch die Steuereinheit, von mindestens einem Aspekt der UV-Lampe als Reaktion auf den Umgebungsluftdruck und die Umgebungslufttemperatur, wobei sich eine Durchbruchschwelle ergibt, die geringer als die Durchbruchspannung ist.

12. Verfahren nach Anspruch 11, wobei die Umgebung eine Innenkabine eines Fahrzeugs ist.

13. Verfahren nach Anspruch 11 oder 12, wobei der mindestens eine Aspekt der UV-Lampe Leistung umfasst, die der UV-Lampe von der Leistungsquelle zugeführt wird.

14. Verfahren nach einem der Ansprüche 11 bis 13, wobei der mindestens eine Aspekt der UV-Lampe den Luftdruck innerhalb der UV-Lampe umfasst und wobei das Modifizieren das adaptive Steuern des Gebläses umfasst, um den Luftdruck innerhalb der UV-Lampe zu variieren.

15. Verfahren nach einem der Ansprüche 11 bis 14, mit einem oder mehreren der Folgenden:

wobei der mindestens eine Aspekt einen Abstand zwischen den Elektroden einschließt;
wobei die UV-Lampe eines oder mehrere von dem Drucksensor, dem Temperatursensor und/oder der Steuereinheit umfasst;
wobei einer oder beide des Drucksensors und/oder des Temperatursensors an einem Gehäuse der UV-Lampe befestigt sind.

**Revendications**

1. Système (100), comprenant :

une lampe à rayonnement ultraviolet (UV) (104) comportant un ou plusieurs émetteurs de lumière UV (106) couplés à des électrodes, dans lequel les émetteurs de lumière UV (106) sont configurés pour émettre une lumière UV (110) à l'intérieur d'un environnement ;
une source d'alimentation (114) couplée à la lampe UV (104), dans lequel la source d'alimentation (114) est configurée pour alimenter la lampe UV (104) ;
un capteur de pression (122) configuré pour détecter une pression d'air ambiant à l'intérieur de l'environnement ;
un capteur de température (126) configuré pour détecter une température d'air ambiant à l'intérieur de l'environnement ; et
une unité de commande (118) configurée pour analyser des données de pression d'air concernant la pression d'air ambiant et des données de température d'air concernant la température d'air ambiant par rapport à une tension de claquage pour la lampe UV (104),
dans lequel l'unité de commande (118) est en outre configurée pour modifier au moins un aspect de la lampe UV (104) en réponse à la pression d'air ambiant et à la température d'air ambiant qui produisent un seuil de claquage qui est inférieur à la tension de claquage.

2. Système (100) selon la revendication 1, dans lequel l'environnement est une cabine interne d'un véhicule.

3. Système (100) selon l'une quelconque des revendications 1 à 2, dans lequel l'unité de commande (118) est en communication avec la source d'alimentation (114), et dans lequel l'au moins un aspect de la lampe UV (104) comprend une puissance fournie à la lampe UV (104) à partir de la source d'alimentation (114).

4. Système (100) selon l'une quelconque des revendications 1 à 3, comprenant en outre un ventilateur (116) couplé à la lampe UV (104), dans lequel l'unité de commande (118) est en communication avec le ventilateur (116), dans lequel le ventilateur (116) est configuré pour souffler de l'air de refroidissement dans la lampe UV (104), dans lequel l'au moins un aspect de la lampe UV (104) comprend une pression d'air à l'intérieur de la lampe UV (104), et dans lequel l'unité de commande (118) est configurée pour commander de manière adaptative le ventilateur (116) afin de faire varier la pression d'air à l'intérieur de la lampe UV (104).

5. Système (100) selon la revendication 4, dans lequel la lampe UV (104) comprend un boîtier (124) ayant une entrée d'air et une sortie d'air, dans lequel le ventilateur (116) est couplé à l'entrée d'air.

6. Système (100) selon la revendication 5, dans lequel l'entrée d'air est plus grande que la sortie d'air.

7. Système (100) selon l'une quelconque des revendications 1 à 6, comprenant en outre un ou plusieurs actionneurs (134) couplés aux électrodes, dans lequel le ou les actionneurs (134) sont configurés pour ajuster l'espacement entre les électrodes, dans lequel l'unité de commande (118) est en communication avec le ou les actionneurs (134), et dans lequel l'au moins un aspect comporte l'espacement entre les électrodes.

8. Système (100) selon l'une quelconque des revendications 1 à 7, dans lequel la lampe UV (104) comporte l'un ou plusieurs parmi le capteur de pression (122), le capteur de température (126) ou l'unité de commande (118).

9. Système (100) selon la revendication 8, dans lequel l'un du capteur de pression (122) ou du capteur de température (126) est fixé à un boîtier (124) de la lampe UV (104).

10. Système (100) selon la revendication 8, dans lequel le capteur de pression (122) et le capteur de température (126) sont tous deux fixés à un boîtier (124) de la lampe UV (104).

11. Procédé, comprenant :

l'analyse, par une unité de commande, d'une pression d'air ambiant détectée par un capteur de pression et d'une température d'air ambiant détectée par un capteur de température, par rapport à une tension de claquage d'une lampe à rayonnement ultraviolet (UV) comportant un ou plusieurs émetteurs de lumière UV couplés à des électrodes, dans lequel les émetteurs de lumière UV sont configurés pour émettre une lumière UV à l'intérieur d'un environnement ; et la modification, par l'unité de commande, d'au moins un aspect de la lampe UV en réponse à la pression d'air ambiant et à la température d'air ambiant qui produisent un seuil de claquage qui est inférieur à la tension de claquage.

12. Procédé selon la revendication 11, dans lequel l'environnement est une cabine interne d'un véhicule.

13. Procédé selon les revendications 11 ou 12, dans lequel l'au moins un aspect de la lampe UV comprend une puissance fournie à la lampe UV à partir de la source d'alimentation.

14. Procédé selon l'une quelconque des revendications 11 à 13, dans lequel l'au moins un aspect de la lampe UV comprend une pression d'air à l'intérieur de la lampe UV, et dans lequel ladite modification comprend la commande adaptative du ventilateur afin de faire varier la pression d'air à l'intérieur de la lampe UV.

15. Procédé selon l'une quelconque des revendications 11 à 14, avec un ou plusieurs de ce qui suit :

   dans lequel l'au moins un aspect comporte un espacement entre les électrodes ;
   dans lequel la lampe UV comporte l'un ou plusieurs parmi le capteur de pression, le capteur de température ou l'unité de commande ;
   dans lequel l'un ou deux du capteur de pression et du capteur de température sont fixés à un boîtier de la lampe UV.

FIG. 1

FIG. 2

EP 4 082 582 B1

```
┌─────────────────────┐        ┌─────────────────────┐
│ Receive a pressure  │        │ Receive a temperature│
│ signal indicative ot│        │ signal indicative at │
│ ambient air pressure│        │ ambient air temperature│
└─────────────────────┘        └─────────────────────┘
          200                            202
```

Analyze ambient air pressure and ambient air temperature in relation to a breakdown voltage  ~204

208

Operate UV lamp in normal fashion

No ← Breakdown threshold ? 206

210

Yes ↓

Modify at least one aspect at the UV lamp

**FIG. 3**

**FIG. 4**

16

FIG. 5

FIG. 6

FIG. 7

FIG. 8

EP 4 082 582 B1

FIG. 9A

FIG. 9B

20

**FIG. 10**

EP 4 082 582 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6787782 B1